# EUROPEAN PATENT SPECIFICATION

(11) **EP 1 545 421 B1**
(45) Date of publication and mention of the grant of the patent: **11.06.2008**
(21) Application number: 03794436.0
(22) Date of filing: 08.07.2003
(51) Int. Cl.: A61F 13/56

(54) **SHAPED ELASTIC EAR**
GEFORMTES ELASTISCHES OHR
PATTES ELASTIQUES PROFILEES

(30) Priority: 03.09.2002 US 233712
(43) Date of publication of application: 29.06.2005
(73) Proprietor: 3M Innovative Properties Company, St. Paul, MN 55133-3427 (US)
(72) Inventor: SWENSON, Douglas A.,, Saint Paul, MN 55133-3427 (US); KIELB, David M.,, Saint Paul, MN 55133-3427 (US); EATON, Bradley W.,, Saint Paul, MN 55133-3427 (US)
(74) Representative: Vossius & Partner
(86) International application number: PCT/US2003/021292
(87) International publication number: WO 2004/021949

(56) References cited:
- EP-A- 0 233 704
- EP-A- 0 704 196
- WO-A-00/37005
- WO-A-00/37009
- WO-A-01/82852
- WO-A-95/16425
- WO-A-97/22319
- WO-A-02/069867
- US-A- 3 800 796
- US-A- 5 496 298
- US-A- 5 807 368
- US-A- 6 159 584

## Description

The present invention relates to an elastic attachment element for use on a disposable article, particularly it relates to an elastic ear portion for use on a disposable article providing elasticity and closure functions.

Disposable absorbent articles such as diapers, training pants or adult incontinent materials are well-known and are provided in a wide variety of configurations. A common configuration makes use of a closure element located on a tab or an ear on one end of the, e.g., diaper or disposable absorbent article which attaches to the opposite end of the disposable absorbent article to provide for adjustable fit and optionally also elasticity. Generally, the attachment element is a rectangular tab, which is attached to one or both corners of the diaper at the back portion, which then attaches to an attachment zone at the front portion of the disposable absorbent article. Generally, one end of the rectangular tab is permanently attached to the diaper with the opposite end of the tab provided with a repositionable fastener element such as a pressure-sensitive adhesive or a mechanical fastener. This repositionable fastener element is mated to a suitable material (from which it can be removably attached) on the opposite front face of the diaper or disposable absorbent article. It is also known that this rectangular fastening tab can be provided with an extensible elastic zone, for example, as disclosed in U.S. Patent No. 3,800,796. The disposable absorbent article can also be rectangular in shape as disclosed in the '796 patent. However, more commonly in today's diapers and like disposable absorbent articles, the diaper is in an hourglass-type shape, as disclosed in U.S. Patent No. 5,368,584. This hourglass shape creates a more form fitting engagement with the waist, leg and the hip area of the wearer. The wider portions of the hourglass shape are at the ends of the disposable article and are commonly referred to as the ear portions. These ear portions can be nonelastic (or inelastic) or provided with an elastic as disclosed in the '584 patent. These ear portions engage the waist at the top of the ear and the hips and top portions of the leg of the wearer at the bottom of the ear. U.S. Patent No. 5,496,298 proposes an alternative construction where the diaper chassis is substantially rectangular and the hourglass shape is formed by an attached ear, which ear is entirely elastic. This is described as providing form-fitting engagement around the waist and leg area of the wearer. The elastomeric ear is provided in a specific shape where the elastomeric ear tapers from a proximal edge to a distal edge. The taper is substantially continuous and in a preferred embodiment the taper is in an arc-type shape. However, this construction requires the use of a large elastic element which does not necessarily provide the preferred elastic forces around the waist area.

WO-A-00/37005 relates to an adjustable garment provided for varying uses with infants, toddlers and adults to contain bodily fluids and the like. The garment employs a large elastic side panel which is completely elastic. Additional inelastic closure devices may be applied to the free end of the elastic side panel.

WO-A-00/37009 relates to a disposable absorbent article including an absorbent chassis and a fastening system that together define a refastenable pant. The refastenable pant includes a pair of elastomeric front side panels extending from the waist opening to each leg opening, a pair of elastomeric back side panels extending from the waist opening to each leg opening, and a pair of refastenable seams extending from the waist opening to each leg opening and positioned between the elastomeric front and back side panels.

EP-A-0 704 196 relates to a fastening tape for a sanitary article for fastening the article on the body of a person. The fastening tape is attached to the article at one of its end portions and provided with a fastening means on one surface of the other end portion. A stretchable elastic portion consists of a sandwich structure of a tape section of a stretchable elastic material secured to one surface of the fastening tape at least at both ends thereof, to bridge a section of the fastening tape.

US-A-5 807 368 relates to an elastic film provided with non-elastic regions and elastic regions formed from a multi-layer film of an elastomeric layer and relatively inelastic layer(s). The film may be applied in disposable products, such as baby diapers and adult incontinent devices.

WO-A-95/16425 relates to an article having a front waistband portion, a rear waistband portion and an intermediate portion interconnecting the front end rear waistband portions and comprises a backsheet layer and a pair of side panels. Each side panel includes a terminal free end region which as a predetermined length dimension thereof. A stress beam section is connected to each of the side panels along its free end region, and the beam section has a relatively high Gurley stiffness value and a length dimension which is at least a significant, substantial percentage of the length of the free end region of the side panel. The fastening mechanism is connected to each of the stress beam sections and arranged to extend laterally from each of the side panels for securing the waistband portions of the article on a wearer.

The present invention is defined by the features of the claims.

### Brief Description of the Drawings

FIGURE 1 illustrates a nonelastic ear element, having the shape of the invention elastic ear element.
FIGURE 2 illustrates a first embodiment of elasticized ear elements according to the present invention.
FIGURE 3 is a side view of the Fig. 2 embodiment.
FIGURE 4 illustrates an elasticized ear of a comparative example of the invention.
FIGURE 5 is an alternative embodiment of the invention of elasticized ear.
FIGURE 6 is a further alternative embodiment of an elasticized ear in accordance with the invention.
FIGURE 7 is a graph of elastic properties of the embodiments depicted in Figs. 2, 4, 5 and 6, as also shown in Example 1, Table 1.
FIGURE 8 is a graph of elastic properties of the embodiments depicted in Figs. 2, 4, 5 and 6, as also shown in Example 2, Table 2.

### Detailed Description

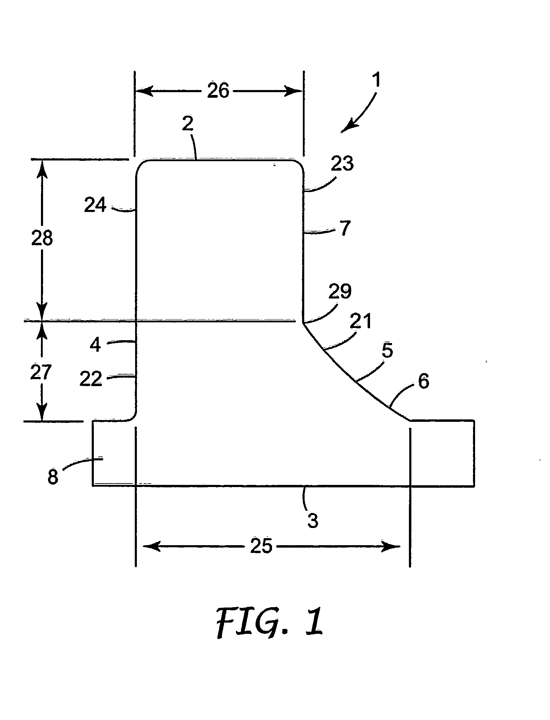

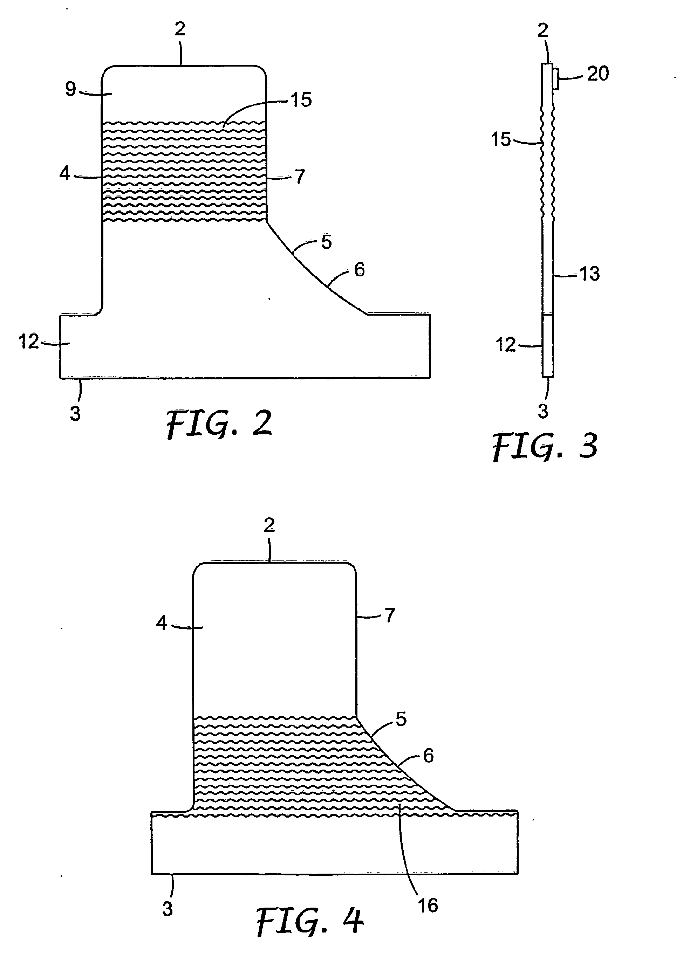

The present invention relates to an attachable ear element 1 which can be attached to the side of a disposable absorbent article for use in closure of the absorbent article. Referring to the Figures, wherein the like numerals represents like elements, Fig. 1 illustrates an ear shape used in the present invention having a free end 2 generally provided with a fastening element 20 and an attached end 3, which is generally attached to the disposable absorbent article. The top edge 4 of the ear element generally defines a portion of the waist opening of the absorbent article when the ear element is being used. The bottom edge 5 of the ear element forms or faces the leg engaging area of the absorbent article. The ear element has two zones. A rectangular zone 7, which generally includes a fastener zone 9 for provision of a fastening element 20, such as a mechanical fastening element such as a hook and/or loop or an adhesive fastening element. Generally, an adhesive fastening element is a suitable pressure-sensitive adhesive or cohesive material. The rectangular zone 7 is also provided at least in part with an elastic region 15 as shown in Fig. 2. The ear element 1 also has a tapered zone 6, which tapered zone is preferably at least in part nonelastic. A tapered zone 6 generally has a tapered edge 21 on the bottom edge 5 of the ear element. Opposite the tapered edge 21 is a top edge 22 of the tapered zone, which preferably is a flat edge contiguous with the top edge 24 of the rectangular zone 7. Rectangular zone 7 has a top edge 24 and a bottom edge 23. The ear element shown in Fig. 1 further is provided with an attachment zone 8 for permanent attachment to the side edge of the disposable absorbent article. This attachment zone can be attached to the disposable absorbent article by use of a conventional attachment method such as ultrasonic, point heat bonding, adhesives or the like. Preferably this attachment zone 8 is nonelastic in its entirety.

It has been discovered that by providing a rectangular zone within an elastic ear element and that the rectangular zone 7 be provided with substantially all the elastic material that a more efficient utilization of elastic material is realized. Specifically there is provided a higher net force per unit area of elastic material while also providing uniform elastic properties over a wide extension range allowing the tabs to be functional over a wide range of user sizes. Generally with the invention attachable elastic ear elements the percentage of the area of the elastic material forming an elastic region in the rectangular zone to the total area of the elastic material forming an elastic region is greater than 25%, preferably greater than 50% and in a most preferred embodiment is 100%. The width 26 of the rectangular zone is generally from 20 to 150 mm and preferably 30 to 100. The end width 25 of the tapered zone 6 is preferably 30 to 180 mm and most preferably 50 to 150 mm where the ratio of the rectangular zone width to the end width of the tapered zone is preferably 1: 1.2 to 1:6 and most preferably 1:1.4 to 1:5. The tapered zone 6 preferably has a tapered edge 21, which tapers in a linear or nonlinear manner and can be arcuate, undulating or the like. If the taper is nonlinear it is preferred that the taper be arcuate and have an inwardly tapered arc as disclosed in U.S. Patent No. 5,496,298, where the taper defines an arc of a circle generally having a radius of from 25 mm to 150 mm and preferably from 50 mm to 90 nim. The tapered zone length 27 will generally be from 10 to 100 mm, preferably 20 to 80 mm whereas the rectangular zone is preferably 10 to 100 mm, most preferably 20 to 80 mm. The ratio of the rectangular zone length to tapered zone length is generally from about 10:1 to 1:10, preferably 2:1 to 1:2. The tapered zone tapered edge 21 preferably tapers continuously into the rectangular zone bottom edge 23 without any rapid discontinuities at transition zone 29. A discontinuity is a rapid change in the width of the ear, over a short distance, such as a change in width of about 5 mm or more over a distance of 3 mm or less. Preferably the width of the tapered zone 6 at the transition zone 29 is identical to the width 26 of the rectangular zone 7 at the transition zone 29.

The elastic material can be any suitable elastic sheet material such as a film elastic or nonwoven elastic or a nonwoven/film elastic laminate. A film elastic can be a blend or a multilayer film elastic with inelastic phases or layers. If there are inelastic phases or layers, the layer thicknesses and the amount of the nonelastic phase needs to be such that the elastic material is still substantially elastic, generally having tensile properties sufficient to maintain a seal to prevent leakage and maintain fit but not cause skin irritation or red marking.

The elastic material can be joined (such as by adhesives, point bonding or other known techniques) to an inelastic substrate web, which preferably is a fibrous layer, which substrate web can be expanded when the elastic material to which the substrate is secured, is stretched. Since the substrate web itself is inelastic, the expandability of the layer is achieved by securing the substrate web to the elastic material such that the length of the substrate web is longer than the length of the elastic in its relaxed state in the area containing the elastic material. Accordingly, it will be possible to expand the substrate web upon stretching of the elastic until the elastic material is stretched to a length equal to the length of the attached substrate web. Stretching beyond this limit will require substantial increase in the stretch force because it would require deformation of the substrate web. The substrate web is generally coextensive with the elastic material and will preferably have an expandability of at least 30% and preferably at least 75%, that is to say that the elastic laminate can be stretched by at least 30% and preferably at least 75% of its length in the relaxed state. Typically, the expandability of the substrate web is between 50 and 400% and most preferably between 75% and 200%.

The substrate web is preferably secured to the elastic material in intervals, i.e., when viewed in the longitudinal direction, portions of the substrate web that are connected to the elastic are alternated with portions of the substrate web that are not connected to the elastic material layer. This may be achieved by corrugating the fibrous layer to form arcuate portions and anchor portions therein and then extruding a molten thermoplastic material thereon that forms the elastic film when cooled as disclosed in U.S. Patent No. 6,159,584. Alternatively, the elastic material and the substrate material may be of equal lengths when laminated or bonded. The laminate can be stretched to permanently deform the substrate in a process such as ringrolling as taught in EP 704 196. A nonwoven substrate web used in connection with the present invention can also be a necked or reversibly necked nonwoven web as described in U.S. Patent Nos. 4,965,122; 4,981,747; 5,114,781; 5,116,662; and 5,226,992. In these embodiments the nonwoven web is elongated in a direction perpendicular to the desired direction of extensibility. When the nonwoven web is set in this elongated condition, it will have stretch and recovery properties in the direction of extensibility.

The elastic material comprises an elastomeric material that exhibits elastomeric properties at ambient conditions, i.e., the material will substantially resume its original shape after being stretched. Preferably, the elastic material will sustain only a small permanent set following deformation and relaxation, which set is preferably less than 30% and more preferably less than 20% of the original 50% to 500% stretch. The elastomeric material can be either pure elastomers or blends with an elastomeric phase or content that will still exhibit substantial elastomeric properties at room temperature. Suitable elastomeric thermoplastic polymers include block copolymers or the like. These block copolymers are described in, for example, U.S. Patent Nos. 3,265,765; 3,562,356; 3,700,633; 4,116,917 and 4,156,673. Particularly useful are styrene/isoprene, styrene/butadiene or ethylenebutylene/styrene block copolymers. These blocks may be arranged in any order including linear, radial, branched or star block copolymers. Other useful elastomeric polymers can include elastomeric polyurethanes, elastomeric ethylene copolymers such as ethylene vinyl acetates, ethylene/propylene copolymer elastomers or ethylene/propylene/diene terpolymer elastomers. Blends of these elastomers with each other or with modifying elastomers are also contemplated.

Viscosity reducing polymers and plasticizers can also be blended with the elastomers such as low molecular weight polyethylene and polypropylene polymers and copolymers, or tackifying resins such as Wingtack^{™}, aliphatic hydrocarbon tackifiers available from Goodyear Chemical Company. Examples of tackifiers include aliphatic or aromatic hydrocarbon liquid tackifiers, polyterpene resin tackifiers, and hydrogenated tackifying resins. Aliphatic hydrocarbon resins are preferred.

Additives such as dyes, pigments, antioxidants, antistatic agents, bonding aids, antiblocking agents, slip agents, heat stabilizers, photostabilizers, foaming agents, glass bubbles, reinforcing fiber, starch and metal salts for degradability or microfibers can also be blended into the elastomeric composition for making the elastic material.

In a preferred embodiment of the present invention, the elastic material is a film comprising an elastomeric core layer of elastomeric material provided on one or both major surfaces with a skin layer. Such a multilayer film can conveniently be produced through co-extrusion. The use of a skin layer is particularly useful on the side of the film where the film is being attached to a substrate layer or another material by adhesives because the skin layer may function as a barrier layer to migration of tackifiers and other low molecular weight species into the elastic core layer and also creates a more stable surface for attachment, particularly when the skin layer is an inelastic material.

The multilayer film can be stretched beyond an elastic limit of these skin layers. The skin layers are generally nontacky materials or blends formed of any semicrystalline or amorphous polynier(s) which are less elastomeric than the elastic core layer, are preferably generally inelastic and will undergo relatively more permanent deformation than the elastic core layer at the percentage that the elastic film is stretched. Preferred elastomeric materials for the core layer are olefinic elastomers, e.g. ethylene-propylene elastomers, ethylene propylene diene polymer elastomers, metallocene polyolefin elastomers or ethylene vinyl acetate elastomers, or styrene/isoprene, butadiene or ethylene-butylene/styrene (SIS, SBS, SEBS) block copolymers, or polyurethanes or blends. Generally, the elastomeric materials used can also be blended with nonelastomeric materials in a weight percent range of 0-70%, preferably 5-50%. High percentages of elastomeric materials in the skin layer(s) generally require use of antiblock and/or slip agents to reduce the surface tack and roll unwind force. Preferably, these skin layers are polyolefinic and are formed predominately of polymers such as polyethylene, polypropylene, polybutylene, polyethylene-polypropylene copolymer, however, these skin layers may also be wholly or partly polyamide, such as nylon, polyester, such as polyethylene terephthalate, or the like, and suitable blends thereof. Generally, the skin layer material following the stretching and recovery of the coextruded elastic is in contact with the elastic core layer material in at least one of three suitable modes; first, continuous contact between the elastic core layer and the microtextured skin layer; second, continuous contact between the layers with cohesive failure of the core layer material under the microtextured skin folds; and third, adhesive failure of the skin layer to the core layer under the microtextured folds with intermittent skin layer to core layer contact at the microtexture fold valleys. Generally, in the context of the present invention, all three forms of skin-to-core contact are acceptable. However, preferably the skin and core layers are in substantially continuous contact so as to minimize the possibility of delamination of the skin layer(s) from the elastic core layer.

Generally, the overall elastic core layer to skin layer(s) thickness ratio of the elastic film will be at least 1.5, preferably at least 5.0 but less than 1000 and most preferably from 5.0 to 200. Generally, the overall caliper of the multilayer elastic film is preferably 25 to 200microns. The addition of the skin layer materials generally tends to reinforce the elastic film material. The skin layers can be sufficiently thin and/or soft so that little or no reinforcement of the elastomeric core layer occurs and the film is elastic in its initial elongation as well as its second and subsequent elongations at suitably low stress elongation forces and low hysteresis loss levels when the elastic is cycled in use (e.g. by dimensional changes caused by breathing).

The substrate web may be a woven material, nonwoven material, knit material, paper, film, or any other continuous media. The substrate web may have a wide variety of properties, such as extensibility, elasticity, flexibility, conformability, breathability, porosity, stiffness, etc. Further, the substrates may include pleats, corrugations or other deformations from a flat planar sheet configuration. The substrate web is preferably a fibrous layer and typically a nonwoven web of thermoplastic polymer fibers. Suitable processes for making the nonwoven web include, but are not limited to, airlaying, spunbond, spunlace, bonded melt blown webs and bonded carded web formation processes.

Spunbond nonwoven webs are made by extruding a molten thermoplastic, as filaments from a series of fine die orifices in a spinneret. The diameter of the extruded filaments is rapidly reduced under tension by, for example, non-eductive or eductive fluid-drawing or other known spunbond mechanisms, such as described in U.S. Patent Nos. 4,340,653; 3,692,618; 3,338,992 and 3,341,394; 3,276,944; 3,502,538; 3,502,763; and 3,542,615. The spunbond web is preferably bonded.

The nonwoven web layer also may be made from bonded carded webs. Carded webs are made from separated staple fibers, which fibers are sent through a combing or carding unit which separates and aligns the staple fibers in the machine direction so as to form a generally machine direction-oriented fibrous nonwoven web. However, randomizers can be used to reduce this machine direction orientation. Once the carded web has been formed, it is then bonded by one or more of several bonding methods to give it suitable tensile properties. One bonding method is powder bonding wherein a powdered adhesive is distributed through the web and then activated, usually by heating the web and adhesive with hot air. Another bonding method is pattern bonding wherein heated calender rolls or ultrasonic bonding equipment are used to bond the fibers together, usually in a localized bond pattern though the web can be bonded across its entire surface if so desired. Generally, the more the fibers of a web are bonded together, the greater the nonwoven web tensile properties.

Airlaying is another process by which fibrous nonwoven webs useful in the present invention can be made. In the airlaying process, bundles of small fibers usually having lengths ranging between about 6 to about 19 millimeters are separated and entrained in an air supply and then deposited onto a forming screen, often with the assistance of a vacuum supply. The randomly deposited fibers are then bonded to one another using, for example, hot air or a spray adhesive.

Alternatively, known melt blown webs or spunlace nonwoven webs or the like can be used to form the fibrous layer of the elastic laminate. Melt blown webs are formed by extrusion of thermoplastic polymers from multiple die orifices, which polymer melt streams are immediately attenuated by hot high velocity air or steam along two faces of the die immediately at the location where the polymer exits from the die orifices. The resulting fibers are entangled into a coherent web in the resulting turbulent airstream prior to collection on a collecting surface. Generally, to provide sufficient integrity and strength for the present invention, melt blown webs must be further bonded such as through air bonding, heat or ultrasonic bonding as described above.

The invention elastic ear element is attached as a separate element and generally joined directly to the disposable absorbent article however it could be joined to an intermediate functional or nonfunctional substrate. The elastic ear element may be joined to the article at any suitable location, however it is preferable joined to the absorbent article at a top edge so that the top edge of the ear element is close to the top edge of the absorbent article.

The substrate web to which the elastic material is joined, on one or both sides, preferably forms both the rectangular zone and the tapered zone. In this way the elastic forces are directly translated from the rectangular zone to the tapered zone without weak areas or hard bands formed by bonding of separate webs to form the different zones. This substrate web can generally have a Gurley stiffness of 1-1000 and is preferably formed of a nonwoven, woven or film, or a continuous laminate thereof.

Further, the substrate web could be reinforced and/or weakened at certain locations to help provide desired flexibility and/or stiffness at certain locations. Methods of weakening the material include scoring, cutting, thinning, bending, heat treating, chemical treating and the like. Methods of reinforcing include heat or chemical treating the material, adding material, and increasing the thickness and the like.

A web can be made extensible by skip slitting as is disclosed in, e.g., International Publication No. WO 96/10481. If the substrate web is desired to be extensible in the area attached to the elastic material, the slits are discontinuous and are generally cut on the web prior to the web being attached to the elastic material. Although more difficult, it is also possible to create slits in the substrate web after the substrate web is laminated to the elastic material. At least a portion of the slits in the nonelastic web should be generally perpendicular (or have a substantial perpendicular vector) to the intended direction of extensibility or elasticity (the at least first direction) of the elastic layer. By generally perpendicular it is meant that the angle between the longitudinal axis of the chosen slit or slits and the direction of extensibility is between 60 and 120 degrees. A sufficient number of the described slits are generally perpendicular such that the overall laminate is elastic. The provision of slits in two directions is advantageous when the elastic laminate is intended to be elastic in at least two different directions.

### Test Methods

### Stress-strain

To measure the stress-strain tensile properties of the webs, a cyclic stress-strain tensile test was performed using an INSTRON Model 1122 constant rate of extension tensile tester. The samples were cut with a razor blade into the shapes shown in Figs. 2, 4, 5 and 6. The dimensions of the rectangular elastic zone were 36mm x 55mm with the 36mm dimension being the dimension that was stretched as the sample was elongated. Both the top and bottom crossheads of the tensile tester were equipped with flat jaws. The crosshead separation was set at 50.8mm. The sample was clamped into the jaws and the crossheads were separated at a rate of 508 mm/minute. The crossheads were stopped for 1 second after an extension of 50mm (139% elongation; based on the length of the elastic zone) was achieved and then returned to the starting point (50.8mm jaw separation or 0% elongation). After again pausing for 1 second, a second extension or upload to a 50mm extension (139% elongation) was performed. After pausing for one second, the crossheads were returned to the starting point to conclude the test. The force in grams for the first pull (upload) was read from the resultant stress-strain chart at 12.5mm extension (35% elongation), 25mm extension (69% elongation), 37.5mm extension (104% elongation) and 50mm extension (139% elongation). The force for the second pull (upload) was read at 25mm and 50mm extension. The force was divided by the actual area of elastic in the cut sample to obtain a 'normalized' tensile in grams/mm².

### Examples

### Example 1

A continuous coextrusion was carried out to prepare a three layer laminate consisting of two outer inelastic skin layers of polypropylene; (5E57 available from Union Carbide Corporation, a subsidiary of the Dow Chemical Company, Danbury, CT) and a core layer of block copolymer rubber, (KRATON D1114PX, available from Kraton Polymers, Belpre, OH). The laminate had an overall thickness of 92 microns (3.6 mils) with skin/core/skin thicknesses of 9/74/9 microns. The laminate was then stretched in the transverse direction at a ratio of about 5:1 and was then annealed by passing over a patterned heated roll (106 degrees Celsius). This resulted in alternating parallel longitudinal bands of inelastic and elastic regions running in the machine direction of the film. The elastic regions had a width of 100 mm and the inelastic regions had a width of 44 mm as measured when the elastic laminate was held in the stretched state.

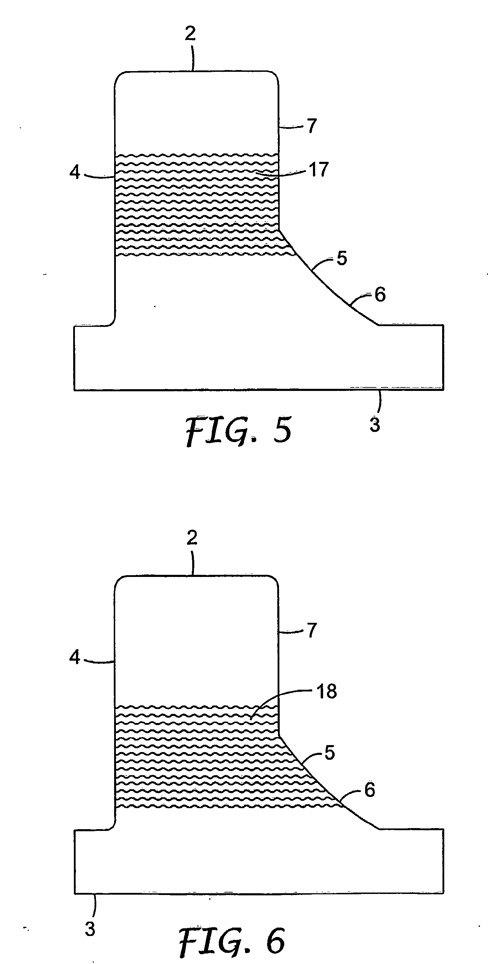

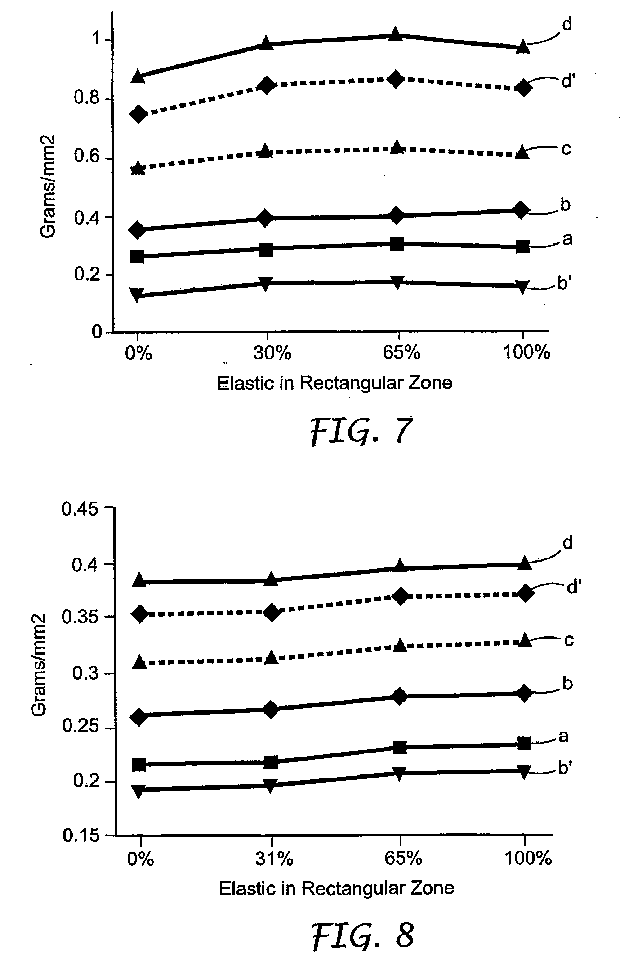

A 15.5 grams/meter² (0.46 ounce/yard²) spunbond polypropylene nonwoven web was adhesively bonded to both the top and bottom side of the elastic laminate while it was held in the stretched state. The nonwoven/elastic/nonwoven laminate was then allowed to relax resulting in a laminate having elastic and inelastic zones. The laminate was then cut into the shape as shown in Figs. 2, 4, 5 and 6. The samples were cut in such a way that 100% of the elastic region 15 was located entirely in the rectangular portion of the shape only (Fig. 2), 65% of the elastic region 17 was located in the rectangular portion of the shape and 35% was located in the trapezoidal portion (Fig. 5), 30% of the elastic region 18 was located in the rectangular portion of the shape and 70% was located in the trapezoidal portion (Fig. 6) and finally where 100% of the elastic region 16 was located in the trapezoidal portion of the shape (Fig. 4). The samples were tested for stress-strain properties with the results shown in Table 1, and summarized in Fig. 7.

### Example 2

The three layer elastic material described in Example 1 above (not laminated to the spunbond nonwovens) was tested for stress-strain properties. The shapes and the location of the elastic within the shape were also the same as described in Example 1 with the exception being that the elastic zone was 28mm x 55mm with the 28mm dimension being the dimension that was stretched as the sample was elongated. The results are shown in Table 2, and summarized in Fig. 8.

**Table 1**

| (Example 1. film/nonwoven laminate) | | | | | | |
|---|---|---|---|---|---|---|
| Shape as shown in: | | Fig. 2 | Fig. 5 | Fig. 6 | Fig. 4 (Comparative) | Fig. 7 Curve |
| Area of elastic in | (%) | 100% | 65% | 30% | 0% | |
| rectangular zone | (mm²) | 1980 | 1320 | 660 | 0 | |
| Amount of elastic in | (%) | 0% | 35% | 70% | 100% | |
| non- rectangular zone | (mm²) | 0 | 700 | 1523 | 2577 | |
| Total Elastic area | (mm²) | 1980 | 2020 | 2183 | 2577 | |
| Distance into rectangular zone | (mm) | 36 | 24 | 12 | 0 | |
| Distance into nonrectangular zone | (mm) | 0 | 12 | 24 | 36 | |
| Tensile - first pull at | (grams) | 594 | 620 | 634 | 673 | a |
| 12.5mm extension | (g/mm²) | 0.300 | 0.307 | 0.290 | 0.261 | |
| Tensile-first pull at | (grams) | 851 | 889 | 921 | 1011 | b |
| 25mm extension | (g/mm²) | 0.430 | 0.440 | 0.422 | 0.392 | |
| Tensile-first pull at | (grams) | 1233 | 1294 | 1353 | 1472 | c |
| 37.5mm extension | (g/mm²) | 0.623 | 0.641 | 0.620 | 0.571 | |
| Tensile-first pull at | (grams) | 1953 | 2063 | 2165 | 2271 | d |
| 50mm extension | (g/mm²) | 0.986 | 1.021 | 0.992 | 0.881 | |
| Tensile - second | (grams) | 337 | 355 | 376 | 391 | b' |
| pull at 25mm extension | (g/mm²) | 0.170 | 0.176 | 0.172 | 0.152 | |
| Tensile - second | (grams) | 1672 | 1775 | 1861 | 1931 | d' |
| pull at 50mm extension | (g/mm²) | 0.844 | 0.879 | 0.853 | 0.750 | |

**Table 2**

| (Example 2; elastic film only) | | | | | | |
|---|---|---|---|---|---|---|
| Shape as shown in: | | Fig. 2 | Fig. 5 | Fig. 6 | Fig. 4 (Comparative) | Fig. 8 Curve |
| Area of elastic in | (%) | 100% | 65% | 31% | 0% | |
| rectangular zone | (mm²) | 1540 | 1029 | 512 | 0 | |
| Amount of elastic in | (%) | 0% | 33% | 67% | 100% | |
| non-rectangular zone | (mm²) | 0 | 554 | 1145 | 1915 | |
| Elastic area | (mm²) | 1540 | 1583 | 1657 | 1915 | |
| Distance into rectangular zone | (mm) | 28 | 9.3 | 18.7 | 0 | |
| Distance into nonrectangular zone | (mm) | 0 | 18.7 | 9.3 | 28 | |
| Tensile - first pull at | (grams) | 358 | 364 | 361 | 415 | a |
| 12.5mm extension | (g/mm²) | 0.233 | 0.230 | 0.218 | 0.217 | |
| Tensile - first pull at | (grams) | 429 | 437 | 441 | 501 | b |
| 25mm extension | (g/mm²) | 0.278 | 0.276 | 0.266 | 0.261 | |
| Tensile - first pull at | (grams) | 501 | 509 | 517 | 594 | c |
| 37.5mtn extension | (g/mm² ) | 0.325 | 0.332 | 0.312 | 0.310 | |
| Tensile - first pull at | (grams) | 608 | 622 | 635 | 731 | d |
| 50mm extension | (g/mm² ) | 0.395 | 0.393 | 0.383 | 0.382 | |
| Tensile - second | (grams) | 319 | 327 | 325 | 369 | b' |
| pull at 25mm extension | (g/mm²) | 0.207 | 0.206 | 0.196 | 0.192 | |
| Tensile-second | (grams) | 567 | 582 | 588 | 677 | d' |
| pull at 50mm extension | (g/mm² ) | 0.368 | 0.368 | 0.355 | 0.353 | |

## Claims

1. An attachable ear element for use on a disposable absorbent article having two opposing ends and a top edge and a bottom edge and a first free end provided with a fastening element and a second opposing attached end, the attachable ear element having a first rectangular zone adjacent the first free end and a second tapered zone adjacent the second attached end, the rectangular zone provided at least in part with an elastic region, wherein the tapered zone is at least in part inelastic, and wherein the percentage of the area of the elastic material forming an elastic region in the rectangular tone to the total area of the elastic material forming an elastic region is greater than 25%.

2. The attachable ear element of claim 1 wherein the fastening element is in the rectangular zone.

3. The attachable ear element of claim 2 wherein the fastening element is in an adhesive attachment.

4. The attachable ear element of claim 2 wherein the fastening element is a mechanical fastening element.

5. The attachable ear element of claim 1 wherein the tapered zone has a tapered edge on an opposing flat edge continuous with the top edge of the rectangular zone.

6. The attachable ear element of claim 1 wherein the attached end is formed by an attachment zone which is inelastic in its entirety.

7. The attachable ear element of claim 1 wherein the percentage area of elastic material, forming an elastic region, in the rectangular zone to elastic materials, forming an elastic region in the tapered zone is greater than 25 percent.

8. The attachable ear element of claim 7 wherein the percentage area of elastic material, forming an elastic region, in the rectangular zone to elastic materials, forming an elastic region in the tapered zone is greater than 35 percent.

9. The attachable ear element of claim 7 wherein the width of the rectangular zone is from 20 to 150 mm and the end width of the tapered zone is 30 to 180 mm and the ratio of the rectangular zone width to the end width of the tapered zone is 1:1.2 to 1:6.

10. The attachable ear element of claim 9 wherein the width of the rectangular zone is from 30 to 100 mm and the end width of the tapered zone is 50 to 150 mm and the ratio of the rectangular zone width to the end width of the tapered zone is 1:1.4 to 1:5.

11. The attachable ear element of claim 5 wherein the tapered edge is linear.

12. The attachable ear element of claim 5 wherein the tapered edge is arcute.

13. The attachable ear element of claim 12 wherein the tapered edge is inwardly tapered or is defined by the arc of a circle having a radius from 25 to 150 mm.

14. The attachable ear element of claim 9 wherein the length of the tapered zone is from 10 to 100 mm and the length of the rectangular zone is from 10 to 100 mm.

15. The attachable ear element of claim 10 wherein the length of the tapered zone is from 20 to 80 mm and the length of the rectangular zone is from 20 to 80 mm.

16. The attachable ear element of claim 15 wherein the ratio of the rectangular zone length to the tapered zone length is 10:1 to 1:10.

17. The attachable ear element of claim 16 wherein the ratio of the rectangular zone length to the tapered zone length is 2:1 to 1:2.

18. The attachable ear element of claim 7 wherein the elastic material is a film elastic.

19. The attachable ear element of claim 18 wherein the film elastic is a coextruded film elastic with an elastomeric core layer and a substantially inelastic skin layer on at least one outer face, which skin layer is attached to an expandable inelastic substrate layer.

20. The attachable ear element of claim 19 wherein the inelastic substrate layer forms both the tapered zone and the rectangular zone.

21. The attachable ear element of claim 20 wherein two expandable substrate layers are provided on each face of the elastic material wherein the inelastic substrate layer is not expandable in areas outside the elastic region.

## Patentansprüche

1. Anbringbares Ohrelement zur Verwendung auf einem wegwerfbaren saugfähigen Gegenstand mit zwei entgegengesetzten Enden und einem oberen Rand und einem unteren Rand und einem ersten freien Ende, das mit einem Befestigungselement und einem zweiten entgegengesetzten angebrachten Ende versehen ist, wobei das anbringbare Ohrelement eine erste rechteckige Zone, die an das erste freie Ende angrenzt, und eine zweite verjüngte Zone, die an das zweite angebrachte Ende angrenzt, aufweist, wobei die rechteckige Zone mindestens teilweise mit einer elastischen Region versehen ist, wobei die verjüngte Zone mindestens teilweise unelastisch ist, und wobei der Prozentsatz des Bereiches des elastischen Materials, das in der rechteckigen Zone eine elastische Region bildet, gegenüber dem Gesamtbereich des elastischen Materials, das eine elastische Region bildet, größer als 25 % ist.

2. Anbringbares Ohrelement nach Anspruch 1, wobei das Befestigungselement sich in der rechteckigen Zone befindet.

3. Anbringbares Ohrelement nach Anspruch 2, wobei das Befestigungselement eine adhäsive Anbringvorrichtung ist.

4. Anbringbares Ohrelement nach Anspruch 2, wobei das Befestigungselement ein mechanisches Befestigungselement ist.

5. Anbringbares Ohrelement nach Anspruch 1, wobei die verjüngte Zone auf einem entgegengesetzten flachen Rand, der mit dem oberen Rand der rechteckigen Zone fortlaufend ist, einen verjüngten Rand aufweist.

6. Anbringbares Ohrelement nach Anspruch 1, wobei das angebrachte Ende durch eine Anbringungszone gebildet ist, die in vollem Umfang unelastisch ist.

7. Anbringbares Ohrelement nach Anspruch 1, wobei der Prozentsatzbereich des elastischen Materials, das in der rechteckigen Zone eine elastische Region bildet, gegenüber elastischen Materialien, die in der verjüngten Zone eine elastische Region bilden, größer als 25 Prozent ist.

8. Anbringbares Ohrelement nach Anspruch 7, wobei der Prozentsatzbereich des elastischen Materials, das in der rechteckigen Zone eine elastische Region bildet, gegenüber elastischen Materialien, die in der verjüngten Zone eine elastische Region bilden, größer als 35 Prozent ist.

9. Anbringbares Ohrelement nach Anspruch 7, wobei die Breite der rechteckigen Zone 20 bis 150 mm beträgt und die Breite des Endes der verjüngten Zone 30 bis 180 mm beträgt und das Verhältnis der rechteckigen Zonenbreite zur Breite des Endes der verjüngten Zone 1:1,2 bis 1:6 beträgt.

10. Anbringbares Ohrelement nach Anspruch 9, wobei die Breite der rechteckigen Zone 30 bis 100 mm beträgt und die Breite des Endes der verjüngten Zone 50 bis 150 mm beträgt und das Verhältnis der rechteckigen Zonenbreite zur Breite des Endes der verjüngten Zone 1:1,4 bis 1:5 beträgt.

11. Anbringbares Ohrelement nach Anspruch 5, wobei der verjüngte Rand linear ist.

12. Anbringbares Ohrelement nach Anspruch 5, wobei der verjüngte Rand bogenförmig ist.

13. Anbringbares Ohrelement nach Anspruch 12, wobei der verjüngte Rand nach innen verjüngt ist oder durch den Bogen eines Kreises mit einem Radius von 25 bis 150 mm definiert ist.

14. Anbringbares Ohrelement nach Anspruch 9, wobei die Länge der verjüngten Zone 10 bis 100 mm beträgt und die Länge der rechteckigen Zone 10 bis 100 mm beträgt.

15. Anbringbares Ohrelement nach Anspruch 10, wobei die Länge der verjüngten Zone 20 bis 80 mm beträgt und die Länge der rechteckigen Zone 20 bis 80 mm beträgt.

16. Anbringbares Ohrelement nach Anspruch 15, wobei das Verhältnis der Länge der rechteckigen Zone zur Länge der verjüngten Zone 10:1 bis 1:10 beträgt.

17. Anbringbares Ohrelement nach Anspruch 16, wobei das Verhältnis der Länge der rechteckigen Zone zur ' Länge der verjüngten Zone 2:1 bis 1:2 beträgt.

18. Anbringbares Ohrelement nach Anspruch 7, wobei das elastische Material eine elastische Folie ist.

19. Anbringbares Ohrelement nach Anspruch 18, wobei die elastische Folie eine coextrudierte elastische Folie ist, die eine Elastomer-Kernschicht und eine im Wesentlichen unelastische Hautschicht auf mindestens einer Außenfläche aufweist, wobei die Hautschicht an einer dehnbaren unelastischen Substratschicht angebracht ist.

20. Anbringbares Ohrelement nach Anspruch 19, wobei die unelastische Substratschicht sowohl die verjüngte Zone als auch die rechteckige Zone bildet.

21. Anbringbares Ohrelement nach Anspruch 20, wobei zwei dehnbare Substratschichten auf jeder Seite des elastischen Materials bereitgestellt sind, wobei die unelastische Substratschicht in Bereichen außerhalb der elastischen Region nicht dehnbar ist.

## Revendications

1. Élément sous forme de patte attachable destiné à une utilisation sur un article absorbant jetable, comportant deux extrémités opposées et un bord supérieur et un bord inférieur et une première extrémité libre pourvue d'un élément de fixation et une deuxième extrémité attachée opposée, l'élément sous forme de patte attachable comportant une première zone rectangulaire adjacente à la première extrémité libre et une deuxième zone amincie adjacente à la deuxième extrémité attachée, la zone rectangulaire étant pourvue au moins en partie d'une région élastique, dans lequel la zone amincie est au moins en partie inélastique, et dans lequel le pourcentage de la surface du matériau élastique formant une région élastique dans la zone rectangulaire par rapport à la surface totale du matériau élastique formant une région élastique est supérieur à 25 %.

2. Élément sous forme de patte attachable selon la revendication 1, dans lequel l'élément de fixation se trouve dans la zone rectangulaire.

3. Élément sous forme de patte attachable selon la revendication 2, dans lequel l'élément de fixation fait partie d'une fixation adhésive.

4. Élément sous forme de patte attachable selon la revendication 2, dans lequel l'élément de fixation est un élément de fixation mécanique.

5. Élément sous forme dé patte attachable selon la revendication 1, dans lequel la zone amincie comporte un bord aminci sur un bord plat opposé dans le prolongement du bord supérieur de la zone rectangulaire.

6. Élément sous forme de patte attachable selon la revendication 1, dans lequel l'extrémité attachée est formée d'une zone de fixation qui est entièrement inélastique.

7. Élément sous forme de patte attachable selon la revendication 1, dans lequel le pourcentage de la surface du matériau élastique formant une région élastique dans la zone rectangulaire, par rapport aux matériaux élastiques formant une région élastique dans la zone amincie, est supérieur à 25 %.

8. Élément sous forme de patte attachable selon la revendication 7, dans lequel le pourcentage de la surface du matériau élastique formant une région élastique dans la zone rectangulaire, par rapport aux matériaux élastiques formant une région élastique dans la zone amincie, est supérieur à 35 %.

9. Élément sous forme de patte attachable selon la revendication 7, dans lequel la largeur de la zone rectangulaire est de 20 à 150 mm et la largeur de l'extrémité de la zone amincie est de 30 à 180 mm et le rapport de la largeur de la zone rectangulaire contre la largeur de l'extrémité de la zone amincie est de 1:1,2 à 1:6.

10. Élément sous forme de patte attachable selon la revendication 9, dans lequel la largeur de la zone rectangulaire est de 30 à 100 mm et la largeur de l'extrémité de la zone amincie est de 50 à 150 mm et le rapport de la largeur de la zone rectangulaire contre la largeur de l'extrémité de la zone amincie est de 1:1,4 à 1:5.

11. Élément sous forme de patte attachable selon la revendication 5, dans lequel le bord aminci est linéaire.

12. Élément sous forme de patte attachable selon la revendication 5, dans lequel le bord aminci est arqué.

13. Élément sous forme de patte attachable selon la revendication 12, dans lequel le bord aminci est effilé vers l'intérieur ou est défini par l'arc d'un cercle ayant un rayon de 25 à 150 mm.

14. Élément sous forme de patte attachable selon la revendication 9, dans lequel la longueur de la zone amincie est de 10 à 100 mm et la longueur de la zone rectangulaire est de 10 à 100 mm.

15. Élément sous forme de patte attachable selon la revendication 10, dans lequel la longueur de la zone amincie est de 20 à 80 mm et la longueur de la zone rectangulaire est de 20 à 80 mm. '

16. Élément sous forme de patte attachable selon la revendication 15, dans lequel le rapport de la longueur de la zone rectangulaire contre la longueur de la zone amincie est de 10:1 à 1:10.

17. Élément sous forme de patte attachable selon la revendication 16, dans lequel le rapport de la longueur de la zoné rectangulaire contre la longueur de la zone amincie est de 2:1 à 1:2.

18. Élément sous forme de patte attachable selon la revendication 7, dans lequel le matériau élastique est un film élastique.

19. Élément sous forme de patte attachable selon la revendication 18, dans lequel le film élastique est un film élastique coextrudé avec une couche centrale élastomère et une pellicule fondamentalement inélastique sur au moins une face externe, ladite pellicule étant fixée à une couche expansible de substrat inélastique.

20. Élément sous forme de patte attachable selon la revendication 19, dans lequel la couche de substrat inélastique forme à la fois la zone amincie et la zone rectangulaire.

21. Élément sous forme de patte attachable selon la revendication 20, dans lequel deux couches de substrat expansibles sont présentes sur chaque face du matériau élastique, la couche de substrat inélastique n'étant pas expansible dans les zones situées en dehors de la région élastique.
